# EUROPÄISCHE PATENTSCHRIFT

(11) **EP 0 967 971 B1**
(45) Veröffentlichungstag und Bekanntmachung des Hinweises auf die Patenterteilung: **12.06.2002**
(21) Anmeldenummer: 98912465.6
(22) Anmeldetag: 11.03.1998
(51) Int. Cl.: A61K 7/50

(54) **KOSMETISCHE ZUBEREITUNGEN**
COSMETIC PREPARATIONS
PREPARATIONS COSMETIQUES

(30) Priorität: 19.03.1997 DE 19711417
(43) Veröffentlichungstag der Anmeldung: 05.01.2000
(73) Patentinhaber: Cognis Deutschland GmbH & Co. KG, 40589 Düsseldorf (DE)
(72) Erfinder: BOYXEN, Norbert, D-47906 Kempen (DE); BEHLER, Ansgar, D-46240 Bottrop (DE); HENSEN, Hermann, D-42781 Haan (DE); SEIPEL, Werner, D-40723 Hilden (DE)
(74) Vertreter: Wacker, Manfred, Dr.
(86) Internationale Anmeldenummer: EP9801395
(87) Internationale Veröffentlichungsnummer: WO9841187

(56) Entgegenhaltungen:
- DE-A- 19 537 836
- DE-A- 19 543 633
- DE-A- 19 605 360
- DE-A- 19 635 553

## Beschreibung

### Gebiet der Erfindung

Die Erfindung betrifft kosmetische Zubereitungen mit rückfettenden Eigenschaften auf Basis von Monoglycerid(ether)sulfaten und Fettsäurepartialglyceriden, ein Verfahren zu ihrer Herstellung durch unterstöchiometrische Sulfatierung von Partialglyceriden sowie die Verwendung der Mischungen zur Herstellung von kosmetischen Mitteln mit rückfettenden Eigenschaften.

### Stand der Technik

Zubereitungen, die zur Reinigung und Pflege der menschlichen Haut und der Haare eingesetzt werden, enthalten in der Regel ein oder mehrere oberflächenaktive Substanzen, insbesondere auf Basis von anionischen oder amphoteren Tensiden. Da die alleinige Verwendung von Tensiden Haut und Haare zu sehr austrocknen würde, ist es im allgemeinen üblich, solchen Mitteln rückfettende Substanzen zuzusetzen. Es liegt dabei auf der Hand, daß diese Stoffe nicht nur eine hinreichende rückfettende Wirkung, sondern den Erfordernissen des Marktes folgend gleichzeitig auch eine optimale dermatologische Verträglichkeit aufweisen müssen.

Aus der Deutschen Patentschrift **DE-C2 4139935** (Kao) sind flüssige Körperreinigungsmittel auf wäßriger Basis bekannt, die 5 bis 35 Gew.-% anionische Tenside, 2,5 bis 15 Gew.-% Alkylpolyglucoside und 0,5 bis 15 Gew.-% gesättigte Fettsäuremonoglyceride mit 8 bis 18 Kohlenstoffatomen im Fettacylrest aufweisen. Die in diesem Dokument vorgeschlagenen Monoglyceride weisen jedoch auch in Abmischung mit Glucosiden eine nicht ausreichende dermatologische Verträglichkeit auf. Zudem sind die Mischungen in Abwesenheit von Wasser in der Regel fest und lassen sich daher nicht kalt verarbeiten. Gegenstand der Europäischen Patentschrift **EP-B1 0554292** (Henkel) sind O/W-Emulsionen, die Ölkörper, Alkylpolyglucoside, Fettsäurepartialglyceride und gegebenenfalls Fettalkohole enthalten. Auch diese Mischungen sind hinsichtlich ihrer rückfettenden Wirkung, ihrer Hautverträglichkeit und Konsistenz in wasserfreiem Zustand nicht vollkommen zufriedenstellend. Aus der Europäischen Patentanmeldung **EP-A1 0538762** (Kao) sind schließlich Haarbehandlungsmittel bekannt, die kationische Tenside, Alkylpolyglucoside und Ölkörper, beispielsweise auch Fettsäuremonoglyceride enthalten.

Die komplexe Aufgabe der Erfindung hat somit darin bestanden, ethylenoxidfreie Rückfettungsmittel zur Verfügung zu stellen, die gleichzeitig bei Raumtemperatur flüssig sind, verdickende Eigenschaften aufweisen und insbesondere eine besonders hohe dermatologische Verträglichkeit aufweisen.

### Beschreibung der Erfindung

Gegenstand der Erfindung sind kosmetische Zubereitungen mit rückfettenden Eigenschaften, enthaltend
(a) Monoglycerid(ether)sulfate und
(b) Fettsäurepartialglyceride.

Überraschenderweise wurde gefunden, daß die erfindungsgemäßen Mischungen eine besonders hohe dermatologische Verträglichkeit bei optimalen rückfettenden Eigenschaften aufweisen. Die Zubereitungen sind zudem bei Raumtemperatur flüssig, so daß sie sich auch kalt verarbeiten lassen. Ein weiterer Vorteil besteht darin, daß die Mischungen in tensidischen Systemen eine Viskosität aufbauen.

### Monoglycerid(ether)sulfate

Monoglyceridsulfate und Monoglyceridethersulfate stellen bekannte anionische Tenside dar, die nach den einschlägigen Methoden der präparativen organischen Chemie erhalten werden können. Üblicherweise geht man zu ihrer Herstellung von Triglyceriden aus, die gegebenenfalls nach Ethoxylierung zu den Monoglyceriden umgeestert und nachfolgend sulfatiert und neutralisiert werden. Gleichfalls ist es möglich, die Partialgfyceride mit geeigneten Sulfatierungsmitteln, vorzugsweise gasförmiges Schwefeltrioxid oder Chlorsulfonsäure umzusetzen [vgl. **EP-B1 0561825, EP-B1 0561999** (Henkel)]. Die neutralisierten Stoffe können - falls gewünscht - einer Ultrafiltration unterworfen werden, um den Elektrolytgehalt auf ein gewünschtes Maß zu vermindern [**DE-A1 4204700** (Henkel)]. Übersichten zur Chemie der Monoglyceridsulfate sind beispielsweise von A.K.Biswas et al. in **J.Am.Oil.Chem.Soc. 37**, **171 (1960)** und F.U.Ahmed **J.Am.Oil.Chem.Soc. 67, 8 (1990)** erschienen. Die im Sinne der Erfindung einzusetzenden Monoglycerid(ether)sulfate folgen der Formel **(I)** 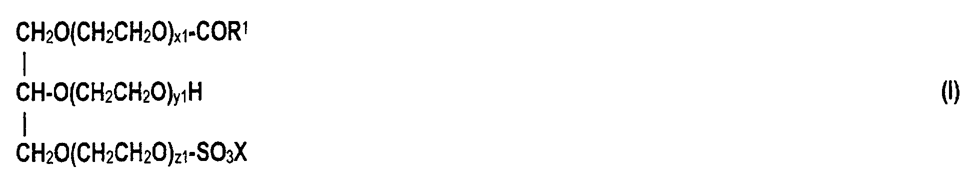 in der R¹CO für einen linearen oder verzweigten Acylrest mit 6 bis 22 Kohlenstoffatomen, x1, y1 und z1 in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel **(I)** eingesetzt, in der R¹CO für einen linearen Acylrest mit 8 bis 18 Kohlenstoffatomen steht.

### Fettsäurepartialglyceride

Bei den Fettsäurepartialglyceriden, die die Komponente (b) bilden, handelt es sich um bekannte Stoffe, die nach den einschlägigen Verfahren der präparativen organischen Chemie hergestellt werden können und der Formel **(II)** folgen, 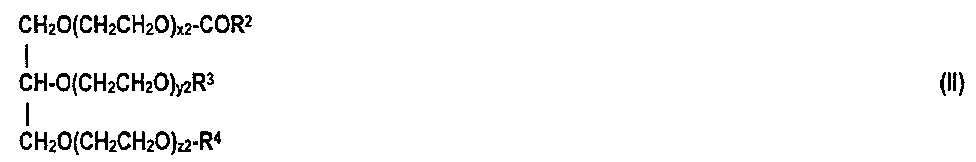 in der R²CO für einen linearen oder verzweigten Acylrest mit 6 bis 22 Kohlenstoffatomen, x2, y2 und z2 in Summe für 0 oder für Zahlen von 1 bis 30, vorzugsweise 2 bis 10, R³ und R⁴ unabhängig voneinander für R²CO oder Wasserstoff steht, mit der Maßgabe, daß R³ und R⁴ nicht beide gleichzeitig eine Acylgruppe darstellen können. Fettsäurepartialglyceride, d.h. in der Regel technische Mischungen von Mono- und Diglyceriden werden üblicherweise durch Umesterung der entsprechenden Triglyceride mit Glycerin oder durch gezielte Veresterung von Fettsäuren erhalten. Die Abtrennung von nichtumgesetzten Ausgangsstoffen sowie die Anreicherung von Monoglyceriden in den Gemischen erfolgt in der Regel über eine Molekulardestillation. Die Partialglyceride der vorliegenden Erfindung werden vorzugsweise durch Veresterung von Glycerin mit Fettsäuren mit 6 bis 22, vorzugsweise 12 bis 18 Kohlenstoffatomen hergestellt wie etwa Capronsäure, Caprylsäure, 2-Ethylhexansäure, Caprinsäure, Laurinsäure, isotridecansäure, Myristinsäure, Palmitinsäure, Paimoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Die Erfindung schließt die Erkenntnis ein, daß technische Mono-/Diglycerid-Mischungen in der Anwendung eine noch bessere dermatologische Verträglichkeit als die reinen Monoglyceride zeigen. Demzufolge sind solche technischen Fettsäuremono-/diglyceride bevorzugt, die ein molares Verhältnis von Mono- zu Diester im Bereich von 10 : 90 bis 90 : 10 und insbesondere 80 : 20 bis 50 : 50 aufweisen.

Mischungen von Kokosmonoglyceridsulfaten und Kokosfettsäuremonoglyceriden zeigen besonders vorteilhafte anwendungstechnische Eigenschaften und sind daher bevorzugt. Die Komponenten (a) und (b) werden in der Regel im Gewichtsverhältnis 30 : 70 bis 99 : 1, vorzugsweise 50 : 50 bis 90 : 10 und insbesondere 70 : 30 bis 80 : 20 eingesetzt.

### Alkyl- und/oder Alkenyloligoglykoside

In einer bevorzugten Ausführungsform der Erfindung enthalten die Zubereitungen als weitere tensidische Inhaltsstoffe Alkyl- und/oder Alkenyloligoglykoside, die der Formel **(III)** folgen,

**R**^{**5**}**O-[G]**_{**p**} **(III)**

in der R⁵ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden, beispielsweise durch säurekatalysierte Acetalisierung von Glucose mit Fettalkoholen. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligo**glucoside**. Die Indexzahl p in der allgemeinen Formel **(III)** gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkylund/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁵ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R⁵ kann sich femer auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

### Ölkörper

Die Zubereitungen können des weiteren Ölkörper enthalten, wie beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, Ester von C₆-C₂₂-Fettalkoholen und/oder Guerbetalkoholen mit aromatischen Carbonsäuren, insbesondere Benzoesäure, pflanzliche Öle, verzweigte primäre Alkohole, substituierte Cyclohexane, lineare C₆-C₂₂-Fettalkoholcarbonate Guerbetcarbonate, Dialkylether, Siliconöle und/oder aliphatische bzw. naphthenische Kohlenwasserstoffe.

### Zubereitungen

Typische rückfettende Zubereitungen, die den Gegenstand der Erfindung illustrieren soll, können beispielsweise eine Zusammensetzung gemäß Tabelle 1 aufweisen:

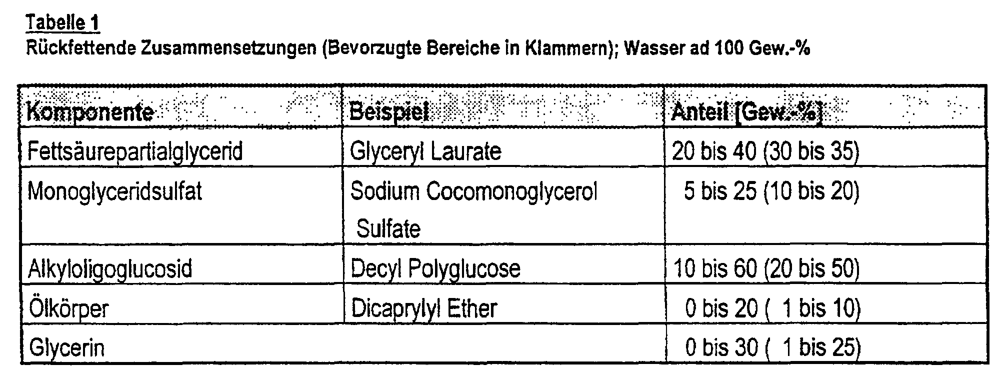

Falls erforderlich, können die erfindungsgemäßen Zubereitungen jeweils 0,5 bis 25 und vorzugsweise 1 bis 15 Gew.-% Glycerin und/oder Wasser enthalten.

### In-situ Herstellung

Die Herstellung der erfindungsgemäßen Zubereitungen kann problemlos durch Vermischen der Komponenten (a) und (b) erfolgen. Eine besonders vorteilhafte Ausgestaltungsform und ein weiterer Gegenstand der Erfindung betrifft jedoch ein Verfahren zur Herstellung von kosmetischen Zubereitungen mit rückfettenden Eigenschaften, bei dem man Fettsäurepartialglyceride mit einer unterstöchiometrischen Menge eines Sulfatiermittels in Kontakt bringt und die resultierenden sauren Sulfatierungsprodukte anschließend mit wäßrigen Basen neutralisiert. In der Praxis bedeutet dies, daß man die Fettsäurepartialglyceride nur anteilig sulfatiert und das Mischungsverhältnis über den Sulfiergrad und damit über die Menge des Sulfatierungsmittels einstellt.

### Sulfatierung und Neutralisation

Die Umsetzung der Partialglyceride mit geeigneten Sulfatierungsmitteln, vorzugsweise gasförmigem Schwefeltrioxid oder Chlorsulfonsäure, kann in der für Fettsäureniedrigalkylester bekannten Weise [**J.Falbe (ed.), "Surfactants in consumer products"; Springer Verlag, Berlin-Heidelberg, 1987, S.61**] erfolgen, wobei Reaktoren, die nach dem Fallfilmprinzip arbeiten, bevorzugt sind. Dabei wird das Schwefeltrioxid mit einem inerten Gas, vorzugsweise Luft oder Stickstoff verdünnt und in Form eines Gasgemisches, welches das Sulfieragens in einer Konzentration von 1 bis 8, insbesondere 2 bis 5 Vol.-% enthält, eingesetzt. Das molare Einsatzverhältnis von Partialglycerid zu Sulfatierungsmittel beträgt - bezogen auf die Hydroxylzahl der Ausgangsstoffe - 1 : 0,3 bis 1 : 0,9, vorzugsweise jedoch 1 : 0,5 bis 1 : 0,85 und insbesondere 1 : 0,7 bis 1 : 0,8. Die Sulfierreaktion wird bei Temperaturen von 35 bis 90, vorzugsweise 35 bis 80°C durchgeführt. Die bei der Sulfatierung anfallenden sauren Sulfierprodukte werden in wäßrige Basen eingerührt, neutralisiert und auf einen pH-Wert von 6,5 bis 8,5 eingestellt. Die Neutralisation wird mit Basen ausgewählt aus der von Alkalimetallhydroxiden wie Natrium-, Kalium- und Lithiumhydroxid, Erdalkalimetalloxiden und -hydroxi-den wie Magnesiumoxid, Magnesiumhydroxid, Calciumoxid und Calciumhydroxid, Ammoniak, Mono-, Di- und Tri-C₂₋₄-Alkanolaminen, beispielsweise Mono-, Di- und Triethanolamin sowie primären, sekun-dären oder tertiären C₁₋₄-Alkylaminen gebildeten Gruppe durchgeführt. Die Neutralisationsbasen ge-langen dabei vorzugsweise in Form 5 bis 55 Gew.-%iger wäßriger Lösungen zum Einsatz, wobei 25 bis 50 Gew.-%ige wäßrige Natriumhydroxidlösung bevorzugt ist. Die Sulfatierungsprodukte können nach der Neutralisation in an sich bekannter Weise durch Zusatz von Wasserstoffperoxid- oder Natriumhypochloritlösung gebleicht werden, um eine für viele Anwendungen erwünschte weitere Farbaufhellung zu erreichen. Dabei werden, bezogen auf den Feststoffgehalt in der Lösung der Sulfierprodukte, 0,2 bis 2 Gew.-% Wasserstoffperoxid, berechnet als 100 Gew.-%ige Substanz, oder entsprechende Mengen Natriumhypochlorit eingesetzt. Der pH-Wert der Lösungen kann unter Verwendung geeigneter Puffermittel, z. B. mit Natriumphosphat oder Citronensäure konstant gehalten werden. Zur Stabilisierung gegen Bakterienbefall empfiehlt sich ferner eine Konservierung, z. B. mit Formaldehydlösung, p-Hydroxybenzoat, Sorbinsäure oder anderen bekannten Konservierungsstoffen.

### Gewerbliche Anwendbarkeit

Die erfindungsgemäßen Zubereitungen zeichnen sich durch eine hohe dermatologische Verträglichkeit und ausgezeichnete rückfettende Eigenschaften aus. Sie sind flüssig und pumpbar und lassen sich daher kalt verarbeiten. Sie sind frei von Stickstoff, gegebenenfalls auch von Ethylenoxid und auch in Abwesenheit von Konservierungsmitteln gegen mikrobiellen Befall ausreichend stabilisiert. Sie weisen verdickende Eigenschaften auf und sind dabei vollständig biologisch abbaubar. Ein weiterer Gegenstand der Erfindung betrifft daher ihre Verwendung als Rückfettungsmittel zur Herstellung von kosmetischen Zubereitungen, beispielsweise für den Bereich der Haar- und Körperpflege.

Die genannten Pflegemittel, wie beispielsweise Haarshampoos, Duschbäder, Schaumbäder, Tagescremes, Nachtcremes, Pflegecremes, Nährcreme, Bodylotions, Salben und dergleichen, können als weitere Hilfs- und Zusatzstoffe milde Tenside, Emulgatoren, Überfettungsmittel, Stabilisatoren, Wachse, Konsistenzgeber, Verdickungsmittel, Kationpolymere, Siliconverbindungen, biogene Wirkstoffe, Antischuppenmittel, Filmbildner, Konservierungsmittel, Hydrotrope, Solubilisatoren, UV-Lichtschutzfilter, Insektenrepellentien, Selbstbräuner, Farb- und Duftstoffe enthalten.

Typische Beispiele für geeignete milde, d.h. besonders hautverträgliche **Tenside** sind Fettalkoholpolyglycolethersulfate, Mono- und/oder Dialkylsulfosuccinate, Fettsäureisethionate, Fettsäuresarcosinate, Fettsäuretauride, Fettsäureglutamate, Ethercarbonsäuren, Fettsäureglucamide, Alkylamidobetaine und/oder Proteinfettsäurekondensate, letztere vorzugsweise auf Basis von Weizenproteinen.

Als **Emulgatoren** kommen beispielsweise nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(3) Anlagerungsprodukte von 15 bis 60 Mol Ethylenoxid an Ricinusöl und/oder gehärtetes Ricinusöl;
(4) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(5) Anlagerungsprodukte von 2 bis 15 Mol Ethylenoxid an Ricinusöl und/oder gehärtetes Ricinusöl;
(6) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(7) Trialkylphosphate sowie Mono-, Di- und/oder Tri-PEG-alkylphosphate;
(8) Wollwachsalkohole;
(9) Polysiloxan-Polyalkyl-Polyether-Copolymere bzw. entsprechende Derivate;
(10) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin,
(11) Polyalkylenglycole sowie
(12) hydrophobierte Polyacrylate.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, und Fettsäurealkanolamide verwendet werden, wobei die letzteren gleichzeitig als Schaumstabilisatoren dienen. Als **Konsistenzgeber** kommen in erster Linie Fettalkohole mit 12 bis 22 und vorzugsweise 16 bis 18 Kohlenstoffatomen und daneben Partialglyceride in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, femer höhermolekulare Polyethylenglycolmono- und -di-ester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. ein quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinyl-imidazol-Polymere wie z.B. Luviquat® (BASF), Kondensationsprodukte von Polyglycolen und Aminen, quaternierte Kollagenpolypeptide wie beispielsweise Lauryldimonium hydroxypropyl hydrolyzed collagen (Lamequat®L/Grünau), quaternierte Weizenpolypeptide, Polyethylenimin, kationische Siliconpolymere wie z.B. Amidomethicone, Copolymere der Adipinsäure und Dimethylaminohydroxypropyldiethylentrimamin (Cartaretine®/Sandoz), Copolymere der Acrylsäure mit Dimethyldiallylammoniumchlorid (Merquat® 550/Chemviron), Polyaminopolyamide wie z.B. beschrieben in der **FR-A 2252840** sowie deren vernetzte wasserlöslichen Polymere, kationische Chitinderivate wie beispielsweise quatemiertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar® C-17, Jaguar® C-16 der Celanese, quaternierte Ammoniumsalz-Polymere wie z.B. Mirapol® A-15, Mirapol® AD-1, Mirapol® AZ-1 der Miranol.

Geeignete **Siliconverbindungen** sind beispielsweise Dimethylpolysiloxane, Methyl-phenylpolysiloxane, cyclische Silicone sowie amino-, fettsäure-, alkohol-, polyether-, epoxy-, fluor- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Carnaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als **Perlglanzwachse** können insbesondere Mono- und Difettsäureester von Polyalkylenglycolen, Partialglyceride oder Ester von Fettalkoholen mit mehrwertigen Carbonsäuren bzw. Hydroxycarbonsäuren verwendet werden. Als **Stabilisatoren** können Metallsalze von Fettsäuren wie z.B. Magnesium-, Aluminium- und/oder Zinkstearat eingesetzt werden. Unter **biogenen Wirkstoffen** sind beispielsweise Tocopherol, Tocopherolacetat, Tocopherolpalmitat, Ascorbinsäure, Retinol, Bisabolol, Allantoin, Phytantriol, Panthenol, AHA-Säuren, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quatemäre Cellulose-Derivate, Kollagen, Hyaluronsäure bzw. deren Salze und ähnliche Verbindungen.

Unter **UV-Lichtschutzfiltern** sind organische Substanzen zu verstehen, die in der Lage sind, ultraviolette Strahlen zu absorbieren und die aufgenommene Energie in Form längerwelliger Strahlung, z.B. Wärme wieder abzugeben. Typische Beispiele sind 4-Aminobenzoesäure sowie ihre Ester und Derivate (z.B. 2-Ethylhexyl-p-dimethylaminobenzoat oder p-Dimethylaminobenzoesäureoctylester), Methoxyzimtsäure und ihre Derivate (z.B. 4-Methoxyzimtsäure-2-ethylhexylester), Benzophenone (z.B. Oxybenzon, 2-Hydroxy-4-methoxybenzophenon), Dibenzoylmethane, Salicylatester, 2-Phenylbenzimidazol-5-sulfonsäure, 1-(4-tert.Butylphenyl)-3-(4'-methoxyphenyl)-propan-1,3-dion, 3-(4'-Methyl)benzylidenbornan-2-on, Methylbenzylidencampher und dergleichen. Weiterhin kommen für diesen Zweck auch feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen, Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C).

Zur Verbesserung des Fließverhaltens können ferner **Hydrotrope** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest wie beispielsweise Methyl- und Butyiglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Als **Konservierungsmittel** eignen sich beispielsweise Phenoxyethanol, Formaldehydlösung, Parabene, Pentandiol oder Sorbinsäure. Als **Insekten-Repellentien** kommen N,N-Diethyl-m-touluamid, 1,2-Pentandiol oder Insect repellent 3535 in Frage, als **Selbstbräuner** eignet sich Dihydroxyaceton. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Beispiele

**Sulfierung von Laurinsäuremonoglycerid.** In einem kontinuierlich arbeitenden Fallfilmreaktor (Länge 120 cm, Querschnitt 1 cm, Eduktdurchsatz 600 g/h) mit Mantelkühlung und seitlicher SO₃-Begasung wurden 1650 g (5 mol) technisches Laurinsäuremonoglycerid (Monomuls 90 L-12) bei 95°C mit Schwefeltrioxid umgesetzt. Das Einsatzverhältnis betrug 0,5 mol SO₃ pro Mol im Partialester enthaltener Hydroxylgruppen. Das Schwefeltrioxid wurde durch Erhitzen aus einer entsprechenden Menge 65 Gew.-%igen Oleums ausgetrieben, mit Stickstoff auf eine Konzentration von 5 Vol.-% verdünnt und über eine Düse mit dem Monoglyceridfilm in Kontakt gebracht. Anschließend wurde es zusammen mit 37 Gew.-%iger Natriumhydroxidlösung in eine 1 Gew.-%igen Lösung von Natriumtriphosphat eingerührt und bei pH = 6,5 bis 8 neutralisiert. Das Reaktionsprodukt wies einen Anteil von 10,9 Gew.-% Laurinsäuremonoglyceridsulfat-Natriumsalz, 7,3 Gew.-% Laurinsäuremonoglycerid, 0,4 Gew.-% Natriumlaurat, 1,5 Gew.-% Natriumsulfat und ad 100 Gew.-% Wasser auf.

**Transepidermaler Wasserverlust.** Zur Beurteilung der dermatologischen Verträglichkeit wurde der transepidermale Wasserverlust an einer Schweineepidermis untersucht. Hierzu wurden definierte Hautstücke bei 40°C über einen Zeitraum von 30 min mit den verschiedenen Testlösungen behandelt und der TEWL-Wert gravimetrisch bestimmt. Die Ergebnisse sind in Tabelle 2 zusammengefaßt. Als TEWL-Wert wird hierbei das in Prozent ausgedrückte Verhältnis des transepidermalen Wasserverlustes einer unbehandelten zu einer behandelten Probe angegeben. Je niedriger der Wert ist, um so besser ist die dermatologische Verträglichkeit. Die Formulierungen 1 bis 4 sind erfindungsgemäß, die Mischungen V1 und V2 dienen zum Vergleich.

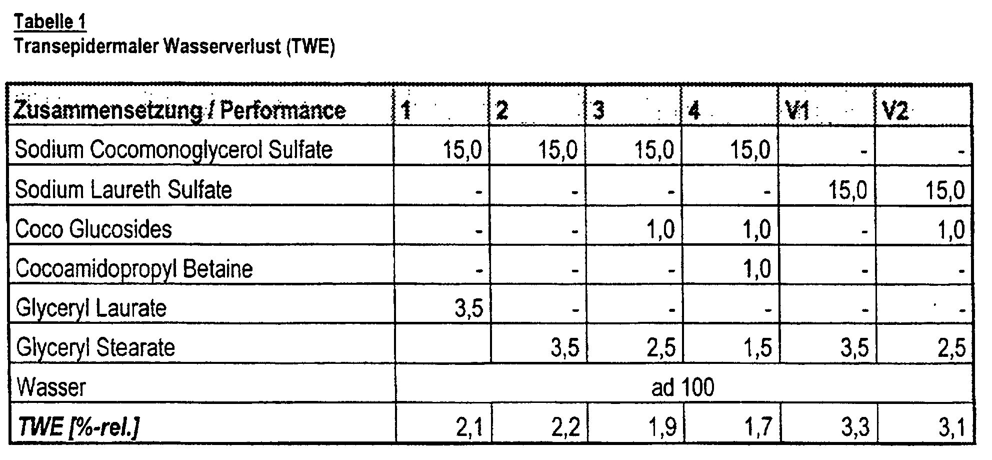

Die Beispiele und Vergleichsbeispiele zeigen deutlich, daß die erfindungsgemäßen Zubereitungen einen wesentlich geringeren transepidermalen Wasserverlust bewirken als die Vergleichsmischungen und somit eine signifikant bessere dermatologische Verträglichkeit besitzen. In Tabelle 3 sind Modellrezepturen für eine Haarspülung (5), verschiedene Duschbäder (6 bis 8) und eine Shampooformulierung (9) angegeben.

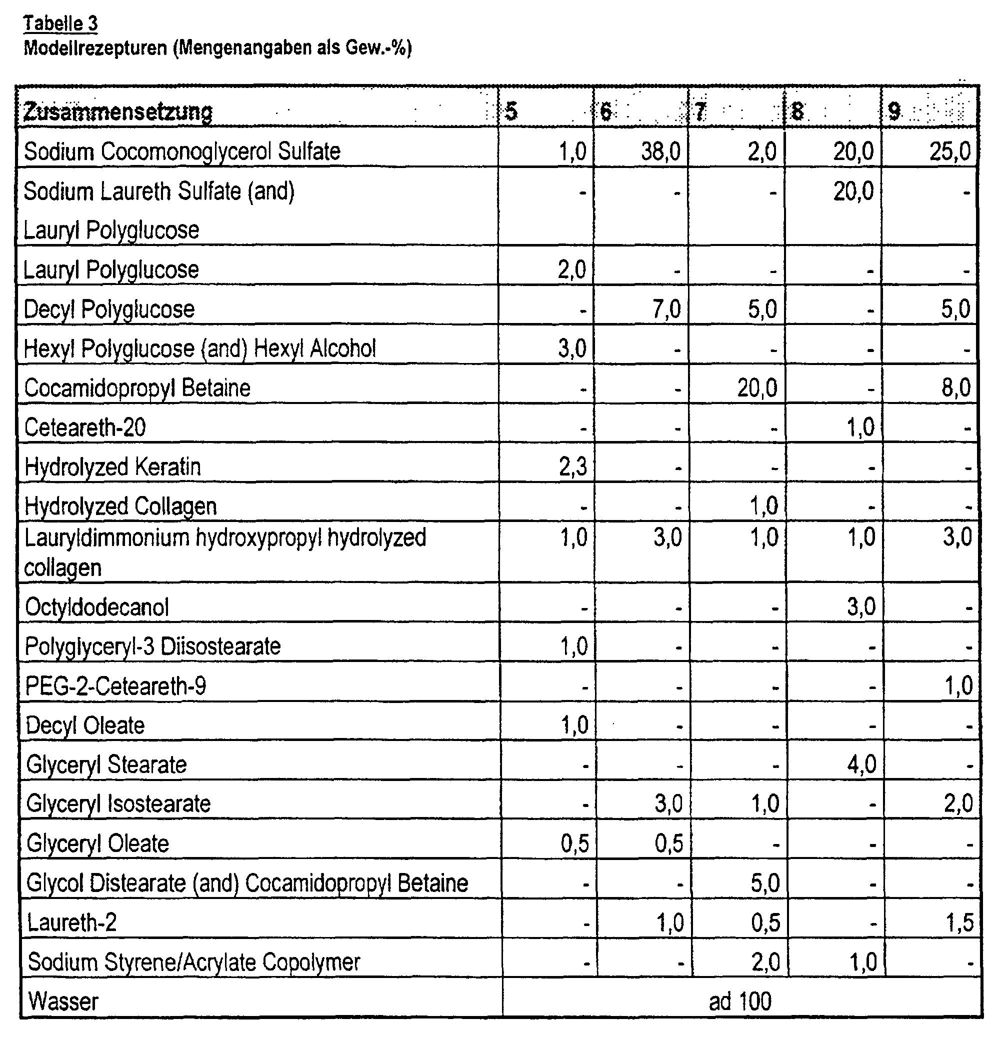

## Patentansprüche

1. Kosmetische Zubereitungen mit rückfettenden Eigenschaften, enthaltend
(a) Monoglycerid(ether)sulfate und
(b) Fettsäurepartialglyceride.

2. Zubereitungen nach Anspruch 1, **dadurch gekennzeichnet, daß** sie Monoglycerid(ether)sulfate der Formel (I) enthalten, 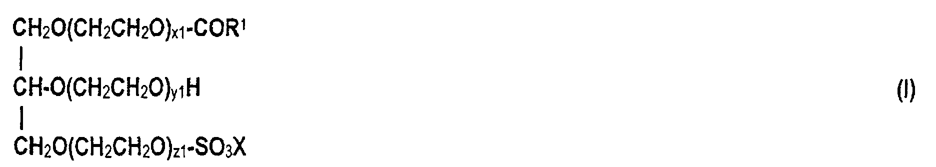 in der R¹CO für einen linearen oder verzweigten Acylrest mit 6 bis 22 Kohlenstoffatomen, x1, y1 und z1 in Summe für 0 oder für Zahlen von 1 bis 30 und X für ein Alkali- oder Erdalkalimetall steht.

3. Zubereitungen nach den Ansprüchen 1 und 2, **dadurch gekennzeichnet, daß** sie Fettsäurepartialglyceride der Formel (II) enthalten, 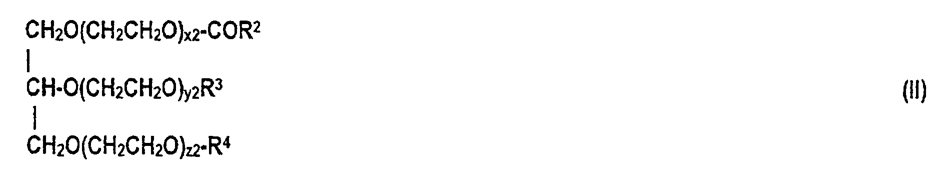 in der R²CO für einen linearen oder verzweigten Acylrest mit 6 bis 22 Kohlenstoffatomen, x2, y2 und z2 in Summe für 0 oder für Zahlen von 1 bis 30, vorzugsweise 2 bis 10, R³ und R⁴ unabhängig voneinander für R²CO oder Wasserstoff steht, mit der Maßgabe, daß R³ und R⁴ nicht beide gleichzeitig eine Acylgruppe darstellen können.

4. Zubereitungen nach den Ansprüchen 1 bis 3, **dadurch gekennzeichnet, daß** sie Mischungen von Kokosmonoglyceridsulfaten und Kokosfettsäuremonoglyceriden enthalten.

5. Zubereitungen nach den Ansprüchen 1 bis 4, **dadurch gekennzeichnet, daß** sie die Komponenten (a) und (b) im Gewichtsverhältnis 30 : 70 bis 99 : 1 enthalten.

6. Zubereitungen nach den Ansprüchen 1 bis 5, **dadurch gekennzeichnet, daß** sie weiterhin Alkylund/oder Alkenyloligoglykoside der Formel (III) enthalten,
**R**^{**5**}**O-[G]**_{**p**} **(III)**
in der R⁵ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht.

7. Zubereitungen nach den Ansprüchen 1 bis 6, **dadurch gekennzeichnet, daß** sie weiterhin Ölkörper enthalten, die ausgewählt sind aus der Gruppe, die gebildet wird von Guerbetalkoholen auf Basis von Fettalkoholen mit 6 bis 18 Kohlenstoffatomen, Estern von linearen C₆-C₂₂-Fettsäuren mit linearen C₆-C₂₂-Fettalkoholen, Estem von verzweigten C₆-C₁₃-Carbonsäuren mit linearen C₆-C₂₂-Fettalkoholen, Estern von linearen C₆-C₂₂-Fettsäuren mit verzweigten Alkoholen, Estern von linearen und/oder verzweigten Fettsäuren mit mehrwertigen Alkoholen und/oder Guerbetalkoholen, Triglyceriden auf Basis C₆-C₁₀-Fettsäuren, Estern von C₆-C₂₂-Fettalkoholen und/oder Guerbetalkoholen mit aromatischen Carbonsäuren, pflanzlichen Ölen, verzweigten primären Alkoholen, substituierten Cyclohexanen, linearen C₆-C₂₂-Fettalkoholcarbonaten, Guerbetcarbonaten, Dialkylethern, Siliconölen und/oder aliphatischen bzw. naphthenischen Kohlenwasserstoffen.

8. Verfahren zur Herstellung von kosmetischen Zubereitungen mit rückfettenden Eigenschaften, **dadurch gekennzeichnet, daß** man Fettsäurepartialglyceride mit einer unterstöchiometrischen Menge eines Sulfatiermittels in Kontakt bringt und die resultierenden sauren Sulfatierungsprodukte anschließend mit wäßrigen Basen neutralisiert.

9. Verfahren nach Anspruch 8, **dadurch gekennzeichnet, daß** man die Fettsäurepartialglyceride und die Sulfatierungsmittel - bezogen auf die Hydroxylzahl des Ausgangsstoffe - im molaren Verhältnis 1 : 0,3 bis 1 : 0,9 einsetzt.

10. Verwendung von Mischungen, enthaltend
(a) Monoglycerid(ether)sulfate und
(b) Fettsäurepartialglyceride
als Rückfettungsmittel zur Herstellung von kosmetischen Zubereitungen.

## Claims

1. Cosmetic preparations with refatting properties containing
(a) monoglyceride (ether)sulfates and
(b) fatty acid partial glycerides.

2. Preparations as claimed in claim 1, **characterized in that** they contain monoglyceride (ether) sulfates corresponding to formula (I): 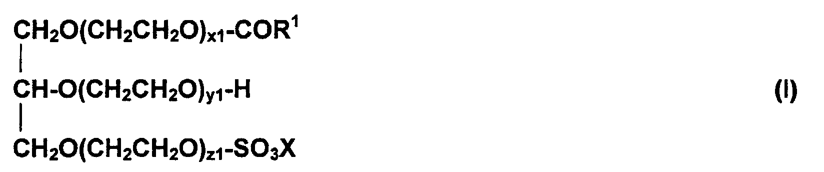 in which R¹CO is a linear or branched acyl group containing 6 to 22 carbon atoms, x1, y1 and z1 together stand for 0 or for numbers of 1 to 30 and X is an alkali metal or alkaline earth metal.

3. Preparations as claimed in claims 1 and 2, **characterized in that** they contain fatty acid partial glycerides corresponding to formula (II): 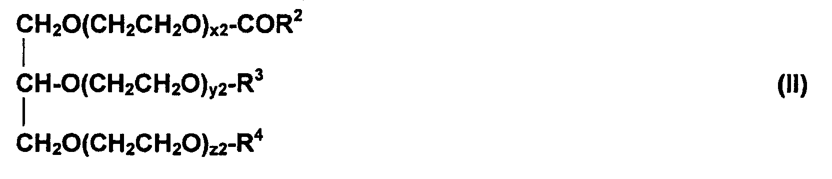 where R²CO is a linear or branched acyl group containing 6 to 22 carbon atoms, x2, y2 and z2 together stand for 0 or for numbers of 1 to 30, preferably 2 to 10, R³ and R⁴ independently of one another have the same meaning as R²CO or represent hydrogen, with the proviso that R³ and R⁴ cannot both be an acyl group.

4. Preparations as claimed in claims 1 to 3, **characterized in that** they contain mixtures of cocomonoglyceride sulfates and cocofatty acid monoglycerides.

5. Preparations as claimed in claims 1 to 4, **characterized in that** they contain components (a) and (b) in a ratio by weight of 30:70 to 99:1.

6. Preparations as claimed in any of claims 1 to 5, **characterized in that** they additionally contain alkyl and/or alkenyl oligoglycosides corresponding to formula **(III)**:
**R**^{**5**}**O-[G]**_{**p**} **(III)**
in which R⁵ is an alkyl and/or alkenyl group containing 4 to 22 carbon atoms, G is a sugar unit containing 5 or 6 carbon atoms and p is a number of 1 to 10.

7. Preparations as claimed in claims 1 to 6, **characterized in that** they additionally contain oils selected from the group consisting of Guerbet alcohols based on fatty alcohols containing 6 to 18 carbon atoms, esters of linear C₆₋₂₂ fatty acids with linear C₆₋₂₂ fatty alcohols, esters of branched C_{6ν13} carboxylic acids with linear C₆₋₂₂ fatty alcohols, esters of linear C₆₋₂₂ fatty acids with branched alcohols, esters of linear and/or branched fatty acids with polyhydric alcohols and/or Guerbet alcohols, triglycerides based on C₆₋₁₀ fatty acids, esters of C₆₋₂₂ fatty alcohols and/or Guerbet alcohols with aromatic carboxylic acids, vegetable oils, branched primary alcohols, substituted cyclohexanes, linear C₆₋₂₂ fatty alcohol carbonates, Guerbet carbonates, dialkyl ethers, silicone oils and/or aliphatic or naphthenic hydrocarbons.

8. A process for the production of cosmetic preparations with refatting properties, **characterized in that** fatty acid partial glycerides are contacted with a less than stoichiometric quantity of sulfating agent and the resulting acidic sulfation products are neutralized with aqueous bases.

9. A process as claimed in claim 8, **characterized in that** the fatty acid partial glycerides and the sulfating agent are used in a molar ratio of 1:0.3 to 1:0.9, based on the hydroxyl value of the starting materials.

10. The use of mixtures containing
(a) monoglyceride (ether) sulfates and
(b) fatty acid partial glycerides
as refatting agents for the production of cosmetic preparations.

## Revendications

1. Préparations cosmétiques ayant des propriétés de regraissage contenant :
a) des (éther) sulfates de monoglycéride, et
b) des glycérides partiels d'acides gras.

2. Préparations selon la revendication 1,
**caractérisées en ce qu'**
elles contiennent des (éther)sulfates de monoglycéride de formule (I) : 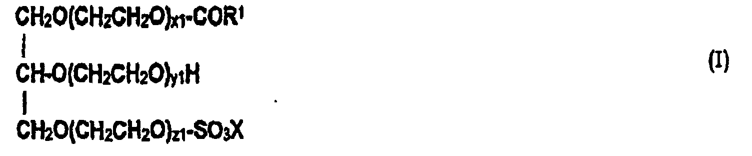 dans laquelle R¹CO représente un reste acyle linéaire ou ramifié ayant de 6 à 22 atomes de carbone, x₁, y₁ et z₁ globalement représentent 0 ou des nombres allant de 1 à 30 et X représente un métal alcalin ou alcalino-terreux.

3. Préparations selon les revendications 1 et 2,
**caractérisées en ce qu'**
elles renferment des glycérides partiels d'acides gras de formule (II), 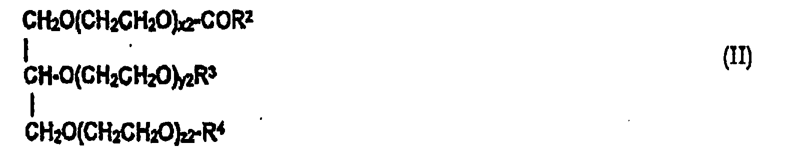 dans laquelle R²CO représente un reste acyle linéaire ou ramifié ayant de 6 à 22 atomes de carbone, x₂, y₂ et z₂ globalement représentent 0 ou des nombres allant de 1 à 30, de préférence de 2 à 10, R³ et R⁴ indépendamment l'un de l'autre, représentent R²CO ou de l'hydrogène, avec la précision que R³ et R⁴ ne peuvent représenter tous les deux en même temps un groupe acyle.

4. Préparations selon les revendications 1 à 3,
**caractérisées en ce qu'**
elles contiennent des mélanges de sulfates de monoglycérides de coco et de monoglycérides d'acides gras de coco.

5. Préparations selon les revendications 1 à 4,
**caractérisées en ce qu'**
elles contiennent les composants a) et b) dans un rapport en poids allant de 30:70 à 99:1,

6. Préparations selon les revendications 1 à 5,
**caractérisées en ce qu'**
elles renferment en outre des alkyl- et/ou alkényl-oligoglycosides de formule (III)
R⁵O-[G]ₚ (III)
dans laquelle R⁵ représente un reste alkyle et/ou alkényle ayant de 4 à 22 atomes de carbone, G représente un reste de sucre ayant 5 ou 6 atomes de carbone et p représente des nombres allant de 1 à 10.

7. Préparations selon les revendications 1 à 6,
**caractérisées en ce qu'**
elles contiennent en outre des composés huileux choisis dans le groupe formé des alcools de Guerbet à base d'alcools gras ayant de 6 à 18 atomes de carbone, des esters d'acides gras en C₆-C₂₂ linéaires avec des alcools gras en C₆-C₂₂ linéaires, des esters d'acides gras linéaires en C₆-C₂₂ avec des alcools ramifiés, des esters d'acide carboxylique en C₆-C₁₃ ramifiés avec des alcools gras en C₆-C₂₂ linéaires, des esters d'acide gras linéaires et/ou ramifiés avec des alcools plurifonctionnels et/ou des alcools de Guerbet, des triglycérides à base d'acides gras en C₆-C₁₀, des esters d'alcools gras en C₆-C₂₂ et/ou d'alcools de Guerbet avec des acides carboxyliques aromatiques, des huiles végétales, des alcools primaires ramifiés, des cyclohexanes substitués, des carbonates d'alcools gras en C₆-C₂₂ linéaires, des carbonates de Guerbet, des éthers dialkyliques, des huiles de silicone et/ou des hydrocarbures aliphatiques ou naphténiques.

8. Procédé de production de préparations cosmétiques ayant des propriété de regraissage,
**caractérisé en ce qu'**
on met en contact des glycérides partiels d'acides gras avec une quantité sous-stoechiomtrique d'un agent de sulfatation et on neutralise les produits acides de sulfatation qui en résultent ensuite avec des bases aqueuses.

9. Procédé selon la revendication 8,
**caractérisé en ce qu'**
on met en oeuvre les glycérides partiels d'acides gras et l'agent de sulfatation, rapporté au nombre d'hydroxyles du composé de départ, dans le rapport molaire de 1:0,3 à 1:0,9.

10. Utilisation de mélanges contenant ;
a) des (ether)sulfates de monoglycéride et
b) des glycérides partiels d'acides gras
en tant qu'agent de regraissage en vue de la production de compositions cosmétiques.
